# EUROPÄISCHE PATENTSCHRIFT

(11) **EP 2 528 660 B1**
(45) Veröffentlichungstag und Bekanntmachung des Hinweises auf die Patenterteilung: **15.04.2015**
(21) Anmeldenummer: 10796084.1
(22) Anmeldetag: 29.12.2010
(51) Int. Cl.: A61Q 5/00, C08G 77/388, C08G 77/46

(54) **NEUARTIGE LINEARE POLYDIMETHYLSILOXAN-POLYETHER-COPOLYMERE MIT AMINO- UND/ODER QUATERNÄREN AMMONIUMGRUPPEN UND DEREN VERWENDUNG**
NOVEL LINEAR POLYDIMETHYLSILOXANE POLYETHER COPOLYMERS HAVING AMINO AND/OR QUATERNARY AMMONIUM GROUPS AND USE THEREOF
NOUVEAUX COPOLYMÈRES LINÉAIRES DE POLYÉTHER POLYDIMÉTHYLSILOXANE COMPORTANT DES GROUPES AMINO ET/OU AMMONIUM QUATERNAIRE ET LEUR UTILISATION

(30) Priorität: 29.01.2010 DE 102010001350
(43) Veröffentlichungstag der Anmeldung: 05.12.2012
(73) Patentinhaber: Evonik Degussa GmbH, 45128 Essen (DE)
(72) Erfinder: SCHUBERT, Frank, 47506 Neukirchen-Vluyn (DE); KNOTT, Wilfried, 45355 Essen (DE); FERENZ, Michael, 45147 Essen (DE); KLEIN, Klaus-Dieter, 45481 Mülheim an der Ruhr (DE); MAURER, Tobias, 42551 Velbert (DE); SCHEUERMANN, Ralph, 45359 Essen (DE); KLUESENER, Bernard William, Harrison, Ohio 45030 (US); PANANDIKER, Rajan Keshav, West Chester, Ohio 45069 (US)
(74) Vertreter: Lang, Arne
(86) Internationale Anmeldenummer: PCT/EP2010/070838
(87) Internationale Veröffentlichungsnummer: WO 2011/091933

(56) Entgegenhaltungen:
- US-A1- 2004 225 099

## Beschreibung

Die Erfindung betrifft neuartige lineare Polydimethylsiloxan-Polyether-Copolymere mit Amino- und/oder quaternären Ammoniumgruppen. Sie betrifft ferner die Verwendung dieser Polymere als Weichmacher für Flächengebilde wie beispielsweise Gewebe, insbesondere textile Gewebe, Tissue, Non-wovens und/oder Fasern aus natürlichen und/oder synthetischen Rohstoffen und/oder Leder, Haar oder Fell sowie deren Verwendung in kosmetischen Anwendungen wie beispielsweise in der Haar-, Haut- und Körperpflege.

Weichmacher für Flächengebilde, wie beispielsweise Gewebe, textile Gewebe, Gewirke, Gestricke, Non-wovens und/oder Fasern aus natürlichen und/oder synthetischen Rohstoffen sind Stoffe, die den genannten Materialien einen weichen geschmeidigen Griff verleihen. Besonders geeignet sind Polysiloxane mit quaternären Ammoniumgruppen. Über elektrostatische Anziehungskräfte verankern die ionischen Gruppen das Siloxan auf der Faser. Auf diese Weise wird die Reibung verringert und der gewünschte Weichmachereffekt erreicht. Trägt man das Siloxan in Form von Mikroemulsionen auf, kann es zudem in die Faser eindringen und verleiht ihr innere Weichheit und Fülle.

Für die Pflege geschädigter Haare gibt es spezielle Zubereitungen, wie Haarspülungen, Haarkuren, Shampoos, Leave-On Konditionierer usw., die vor allem die Kämmbarkeit, den Griff und den Glanz geschädigter Haare verbessern. Derartige handelsübliche Haarpflegemittel enthalten hauptsächlich kationische Tenside auf Alkylammonium-Basis, Polymere, Wachse bzw. Öle oder Siliconöle. Die Wirksamkeit dieser Verbindungen lässt sich u.a. auf eine Hydrophobierung der Haaroberfläche zurückführen.

Bei allen diesen Mitteln wird zwar eine gute Pflegewirkung (Konditionierung) der Haare erreicht, aber das Aussehen, insbesondere der Glanz der Haare, wird durch die Pflegeprodukte nicht verbessert, sondern teilweise sogar verschlechtert.

Es besteht daher also ein Bedarf an vielseitig einsetzbaren Wirkstoffen für Körperreinigungs- und Pflegemittel wie Shampoos, Haarbehandlungsmittel und Haarnachbehandlungsmittel, die neben der reinigenden Wirkung die Pflege des Haares verbessern und gleichzeitig guten Glanz verleihen, die das Haar vor der Schädigung der Haarstruktur schützen und die bereits verursachten strukturellen Schädigungen des Haares, hervorgerufen durch Umwelteinflüsse sowie durch form- und farbgebende Behandlungen, minimieren.

Polysiloxane mit quaternären Ammoniumgruppen sind als Additive für Haarpflege bekannt. So werden zum Beispiel in DE 14 93 384, EP 0 017 122 und US 4,895,964 Strukturen beschrieben, bei denen Siloxane mittelständig mit statistisch über das Polymer verteilten Ammoniumgruppen modifiziert sind. Diese Verbindungen haben den Nachteil, dass sie keinen ausgeprägten Siliconcharakter besitzen und eine gute Wirksamkeit nicht mehr zu beobachten ist.

Einen ausgeprägteren Siliconcharakter haben kationische Polysiloxane, wie sie in DE 37 19 086 und EP 0 294 642 beschrieben werden. Bei den in der DE 37 19 086 und den in der EP 0 294 642 beschriebenen Strukturen sind die quaternären Funktionen endständig an das Polysiloxan gebunden. Derartige Verbindungen bieten Vorteile hinsichtlich ihrer Wirkung als Konditioniermittel sowohl für Haare und Textilien als auch für harte Oberflächen. Die Verwendung solcher Verbindungen in kosmetischen Formulierungen ist z.B. in EP 0 530 974, EP 617 607, EP 1 080 714, WO 2001/082879 und US 6,207,141 beschrieben worden.

Allerdings besitzen die dort beschriebenen Strukturen nur zwei kationische Gruppen. Aufgrund der relativ geringen elektrostatischen Wechselwirkung der nur mit zwei an ihren Termini befindlichen Ladungszentren ausgestatteten Polysiloxane ist deren Affinität zu bestimmten Oberflächen und in deren Folge auch deren Substantivität, d.h. ihre Neigung sich auf denselben dauerhaft zu verankern, verhältnismäßig gering.

Polysiloxane mit seitenständig statistisch über das Polymer verteilten quaternären Ammoniumgruppen und deren Anwendung als Textilweichmacher sind zum Beispiel in der DE-AS 14 93 384 beschrieben. Diese Verbindungen haben den Nachteil, dass sie keinen ausgeprägten Siliconcharakter besitzen und eine gute Wirksamkeit als Textilweichmacher nicht zu beobachten ist.

Einen deutlich stärker ausgeprägten Siliconcharakter haben dagegen kationische Silicone, wie sie in der EP 0 294 642 beschrieben werden. Die EP 0 294 642 beschreibt Strukturen, bei denen die quaternären Funktionen endständig an ein Siloxansegment gebunden sind. Wird ein Textil mit derartigen Verbindungen behandelt, so erhält es zwar einen guten Weichgriff, jedoch ist das Siloxan aufgrund seiner geringen Substantivität leicht wieder von dem entsprechendem Textil entfernbar, zum Beispiel durch Waschvorgänge. Im Gegensatz zum Haushaltsweichspüler ist es für die industrielle textile Endausrüstung jedoch wünschenswert, dass das Siloxan auch nach der Wäsche auf dem Textil verbleibt und der Weichgriff somit nicht verloren geht.

Den Aspekt einer erhöhten Hydrophilie adressierend, beanspruchen die U S 5,807,956 und US 5,981,681 nicht-hydrolysierbare Blockcopolymere des (AB)ₙA-Typs mit alternierenden Einheiten bestehend aus Polysiloxan und Amino-Polyalkylenoxid und einen Weg zu deren Herstellung. So werden durch Edelmetall-katalysierte Hydrosilylierung α,ω-Dihydrogenpolydimethylsiloxane mit Epoxidgruppen tragenden Olefinen SiC-mäßig verknüpft und die so erhaltenen, Epoxy-terminierten Siloxane mit Amino-terminierten Polyalkylenoxiden zur Umsetzung gebracht. Alternativ wird auch auf die hydrosilylierende Verknüpfung von α,ω-Dihydrogenpolydimethylsiloxanen mit epoxy-terminierten Allylpolyethern und die nachfolgende Umsetzung der so erhaltenen epoxy-funktionalisierten Siloxane mit Diaminen abgestellt.

Die Lehre der WO 02/092904 bezieht sich auf Zusammensetzungen bestehend aus nicht-hydrolysierbaren, blockartigen Copolymeren, die keine (AB)ₙ-Struktur besitzen und die gewonnen werden durch die Reaktion zwischen Epoxidgruppen aufweisenden Polydimethylsiloxanen A und Epoxidtermini tragenden Polyalkylenoxiden B in Gegenwart von primären Aminen und/oder Mischungen bestehend aus primären und sekundären Aminen. Die Präsenz unterschiedlicher Epoxidsubstrate führt in Gegenwart der aminischen Reaktanden zu einem praktisch nicht steuerbaren, selbstorganisierten Polyadditionsprozeß, der jenseits einer nicht erzielbaren strengen (A(Amin)B)ₙ-Struktur zu einem Copolymer führt, das in wechselnder Population sowohl homogen-durchsetzte (A(Amin)A) und (B(Amin)B) aber auch heterogen-durchsetzte jeweils aminisch verknüpfte Diaden (A(Amin)B) aufweist. Den so gewonnenen Copolymerstrukturen Ist aufgrund der mit Epoxidfunktionen ausgerüsteten Siloxanedukte eine Verknüpfung der jeweiligen Siloxanyleinheiten über eine Etherfunktion mit den aminoorganischen Resten des Copolymers zu eigen. Mit der weitergehenden Option zur Neutralisation und/oder Quaternisierung lässt sich die Substantivität auf textilen oder faserigen Substraten einstellen. Nachteilig für die Anwendung ist die diesen Random-Copolymerstrukturen eigene Intrinsisch hohe Viskosität.

Die US 2004/225099 A1 offenbart ein Copolymer aus Amlnosiloxan und Polyether, erhältlich durch Umsetzung von Aminosiloxan und einem Addukt aus Epoxid und Amin und die Verwendung solcher Copolymere in Shampoos.

Die dargelegten Nachteile des Standes der Technik erkennend liegt dieser Erfindung die Aufgabe zugrunde, neuartige Copolymere auf Basis von Polysiloxanen mit Amino- und/oder quaternären Ammoniumgruppen zu finden, welche bei guter synthetischer Zugängilchkelt eine Vielzahl gezielt determinierbarer Strukturen und damit auch weit einstellbarer Eigenschaftsprofile ermöglichen.

Abseits des in der Lehre der WO 02/092904 akzeptierten Zufallsprinzips soll der Aufbau klar sequenzierter Copolymerstrukturen, die sowohl aminische respektive Ammonium-Funktionen, Siloxansegmente als auch Polyoxyalkylensegmente umfassen, ermöglicht werden. Das Aufbauprinzip der Copolymere soll darüber hinaus die Option bieten, den Gehalt an inkorporiertem Stickstoff in gewissen Grenzen frei zu wählen.

Gelöst wird die erfindungsgemäße Aufgabe durch neuartige lineare Polydimethylsiloxan-Polyether-Copolymere der Formel (4).

Gegenstand der Erfindung sind daher neuartige lineare Polydimethylsiloxan-Polyether-Copolymere mit Amino- und/oder quaternären Ammoniumgruppen gemäß Formel (4), die erhältlich sind durch die Umsetzung von mit sekundären Amlnoalkylgruppen funktionalisierten Organopolysiloxanen mit den Reaktionsprodukten aus Epoxidgruppen enthaltenden Verbindungen und Aminen.

Die Affinität zu dem textilen und/oder faserigen Träger und letztlich hierdurch bedingt die Substantivität der erfindungsgemäß beanspruchten Copolymerstruktur auf demselben wird durch die definierte Distanz der in elektrostatische Wechselwirkung eintretenden Stickstofffunktionen mit der Oberfläche des Substrats determiniert. Anders ausgedrückt, führt der erfindungsgemäß beanspruchte Syntheseweg zu strukturell ausgewogenen Weichmacheradditiven, die weder eine unerwünschte Häufung von Siloxanfunktionen, noch von aminoorganischen Funktionen aufweisen. Hierdurch unterscheidet sich das hier beanspruchte Verfahren zur Herstellung und das sich hieraus ergebende Copolymer von den Copolymerzusammensetzungen gemäß der Lehre von WO 02/092904, die sogar als Extremfallbetrachtung rein siliciumorganische (A(Amin)A)- und rein organische (B(Amin)B)-Verbindungen enthalten können. Die Präsenz dieser von diametral entgegengesetzter Polarität gekennzeichneten Verbindungen gibt Anlass zu unerwünschten Trübungen und Entmlschphänomenen.

Insbesondere erweist sich die In US 5 486 634 aufgezeigte Methode zur Herstellung der mit sekundären Amlnoalkylgruppen funktlonaüsierten Organopolysiloxane als günstiger Zugang für diese Edukte.
a) ein Diepoxid mit mindestens einer Aminverbindung zu einem endständig Glycidylgruppen tragenden kettenverlängerten Addukt umgesetzt wird, wobei das molare Verhältnis von Diepoxid zu Amin variabel aber mindestens größer 1 zu 1 ist, und
b) dieses Zwischenprodukt anschließend mit einem terminal sek. Aminogruppen tragenden linearen Polysiloxan zur Reaktion gebracht wird,
c) optional zur vollständigen oder teilweisen Quaternierung der Stickstoffatome an beliebiger Stelle in diesem Verfahren, Säuren oder Alkylierungsreagentien zugefügt werden können, und
d) weiterhin optional Wasser oder organische Verbindungen ausgewählt aus der Gruppe enthaltend Polyether, Polyole oder Alkohole als Verdünnungsmittel, Emulgatoren oder Modifizierungsmittel zugefügt werden.

Als Epoxidkomponente im Sinne dieses erfindungsgemäßen Verfahrens werden vorzugsweise Diepoxide oder Diglycidylether der Formel (1) eingesetzt, 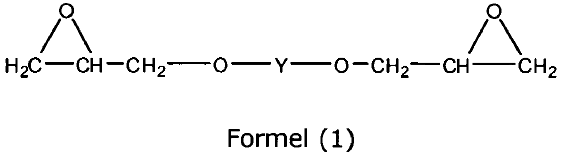 wobei
- Y: ein beliebiger divalenter organischer Rest, vorzugsweise ein divalenter Kohlenwasserstoffrest ist, der durch Sauerstoffatome unterbrochen sein kann. Insbesondere ist Y ein divalenter Polyetherrest des Typs - (CH₂ - CHR² - O)ₘ-CH₂ -CHR² - und m eine ganze Zahl von 0 bis 50 und R² Wasserstoff oder eine Alkylgruppe mit 1-4 C-Atomen.
- Y: Y ein divalenter Polyetherrest des Typs - (CH₂ - CHR² - O)ₘ-CH₂ -CHR² - und m eine ganze Zahl von 0 bis 50 und R² Wasserstoff oder eine Alkylgruppe mit 1-4 C-Atomen ist.

Besonders geeignet sind Diglycidylether von Polypropylenglykolen und Polyethylenglykolen, wie sie z.B. von DOW (D.E.R^{®} 732, D.E.R^{®} 736) und DOW Epoxy Systems (Polypox^{®} R19 - alle genannten Warenzeichen sind Zeichen der DOW Chemical Corp.) erhältlich sind. Auch können verschiedene Diepoxide gemäß Formel (1) Im Gemisch eingesetzt werden.

Als Reaktionspartner für solche Diglycidylether eignen sich prinzipiell alle Aminverbindungen mit zwei reaktiven am Stickstoff gebundenen Wasserstoffatomen. Erfindungsgemäß eingesetzt werden di-sekundäre Amine ausgewählt aus Piperazin, N,N'-Dimethylhexamethylen-1,6-diamin und N,N'-Dimethyltetramethylen-1,4-diamin.

Im Sinne der vorliegenden Erfindung wird mindestens eine Diepoxidkomponente der Formel (1) im molaren Überschuss mit mindestens einer Aminverbindung ausgewählt aus Piperazin, N,N'-Dimethylhexamethylen-1,6-diamln und N,N'-Dimethyltetramethylen-1,4-diamin zu einem endständig Glycidylgruppen tragenden kettenverlängerten Addukt umgesetzt. Das molare Verhältnis von Diepoxid zu Amin ist variabel und ist mindestens größer als 1:1 und beträgt vorzugsweise 10 zu 1 bis 1,1 zu 1 und insbesondere 6 zu 1 bis 1,5 zu 1. Je niedriger der Dlepoxidüberschuss, desto höher ist der Wert von Index x in Formel (6) und die Kettenlänge des resultierenden Addukts. Je höher der Diepoxidüberschuss, desto mehr unadduktierte Diglycidylverbindungen liegen im entstehenden Reaktionsgemisch vor. In jedem Fall stellt das Reaktionsprodukt ein Gemisch von Addukten mit einer Molmassenverteilung dar. Die Umsetzung kann im Temperaturbereich von 20-180 °C, bevorzugt bei 50 bis 120°C durchgeführt werden. Am günstigsten Ist es, die mengenmäßig überwiegende Komponente - zumeist das Diepoxid - vorzulegen und die Zwettkomponente - In der Regel das Amin - unter Rühren und ggfs. Kühlen hinzuzugeben. Es ist möglich, aber meist nicht erforderlich, die Reaktion in einem Lösungsmittel durchzuführen. Sollte dies z.B. wegen hoher Viskosität erforderlich sein, so eignen sich z.B. Ethanol, Propanol, Isopropanol, Butanol, THF, Aceton, Toluol, etc. Wird die Reaktion unter Inertbedingungen wie unter Stickstoff ausgeführt, werden farblich helle, oder auch leicht gelbliche Endprodukte erhalten.

Zur Herstellung der erfindungsgemäßen Copolymere werden lineare, terminal sekundäre Amingruppen aufweisende Polysiloxane gemäß Formel (3) verwendet, 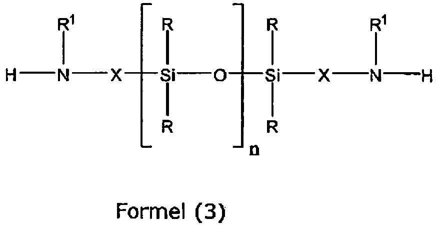 wobei
- R: eine Methylgruppe darstellt,
- R¹: ein beliebiger Kohlenwasserstoffrest ist, bevorzugt ein Alkylrest mit 1 bis 20 C-Atomen, besonders bevorzugt ein Alkylrest mit 1 bis 4 C-Atomen, insbesondere eine Methyl- oder Ethylgruppe Ist,
- X: ein divalenter linearer oder verzweigter Kohlenwasserstoffrest mit 1-20 C-Atomen ist, welcher durch Stickstoffatome bzw. aminische Gruppen unterbrochen sein kann,

Die Umsetzung zwischen mindestens einem Aminosiloxan der Formel (3) und mindestens einem Diepoxid-Amin-Addukt geschieht vorzugsweise in einem Lösemittel wie Isopropanol, Ethanol, Propanol oder THF, um die beiden Reaktionspartner zu kompatibilisieren. Die Stöchiometrie ist grundsätzlich beliebig, bevorzugt werden jedoch beide Komponenten in annähernd äquimolarem Verhältnis zur Reaktion gebracht. Die Relation von sek. Amingruppen aus dem Siloxan der Formel (3) zu den Epoxidgruppen des Diepoxid-Amin-Addukts beträgt vorzugsweise 1,5:1 bis 0,7:1, besonders bevorzugt 1,3:1 bis 0,9:1. Je nachdem, welche der beiden Reaktanden evtl. im Überschuss eingesetzt wird, entstehen linear aufgebaute Copolymere mit endständigen Epoxid- oder Amingruppen. Zur Erzielung lagerstabilerer Produkte ist es von Vorteil, das Aminosiloxan in einem leichten Überschuss gegenüber der Diepoxid-Amin-Adduktkomponente einzusetzen. Die Umsetzung geschieht bei Temperaturen zwischen 20 °C und 180 °C, vorzugsweise bei 60 °C bis 120 °C. Dabei kann wahlweise die Siloxanverbindung vorgelegt und das Diepoxid-Amin-Addukt zugefügt oder in der Dosierreihenfolge umgekehrt verfahren werden. Das Lösemittel kann am Ende im Produkt verbleiben oder bei Bedarf durch dem Fachmann bekannte Verfahren, beispielsweise destillativ, entfernt werden. An beliebiger Stelle im Herstellverfahren, am günstigsten nach Ende der vorbeschriebenen Umsetzung im noch Lösemittel enthaltenden System, können optional Säuren, vorzugsweise Carbonsäuren wie Ameisen-, Essig-, Propion-, Milch-, Oxal-, Zitronen- oder Weinsäure, aromatische Carbonsäuren wie Benzoe- oder Salicylsäure, ebenso aber auch anorganische Säuren wie Phosphor-, Schwefel-, Toluolsulfon-, Methansulfon- oder Salzsäure zugefügt werden, wodurch quaternäre Ammoniumgruppen ausgebildet werden. Die Einsatzmenge der Säure bezogen auf Stickstoff ist variabel und beträgt vorzugsweise zwischen 0,01 mol und 2 mol, bevorzugt 0,1 bis 1,5 mol pro N-Atom.

Im Sinne der erfinderischen Lehre ist es gleichfalls möglich, das nicht-neutralisierte Copolymer zu isolieren und danach, wie zuvor ausgeführt, zu neutralisieren oder eine Quaternisierung der Aminogruppen vorzunehmen. Bei der Quaternisierung der Aminfunktionen werden üblicherweise Alkylierungsreagentien wie Alkylhalogenide oder Alkylsulfate (z.B. Dimethylsulfat) eingesetzt. Einhergehend mit der Quaternisierung gewinnt das Copolymer im Vergleich zum unmodifizierten aminischen Precursor oder dessen Salzen an Substantivität auf textilen oder faserigen Substraten und auch an Einfluss auf deren Neigung zur elektrostatischen Aufladung.

Das erfindungsgemäße Verfahren erlaubt es auf einfache Weise, streng lineare Copolymerstrukturen aufzubauen, in denen sich Siloxaneinheiten und aminfunktionelle organische Sequenzen streng regelmäßig alternierend aneinanderreihen. Der hydrophobe Siloxancharakter kann durch die Siloxankettenlänge, der hydrophile Charakter sowie der Amingehalt durch die Wahl der Diepoxid-Amin-Stöchiometrie im Vorprodukt fast nach Belieben eingestellt werden. So lassen sich reproduzierbar und in der Abfolge der Wiederholeinheiten in der Copolymerkette genau definierte Strukturen herstellen.

Ein Gegenstand der Erfindung ist daher ein Verfahren zur Herstellung von neuartigen linearen Copolymerstrukturen, bestehend aus alternierend abfolgenden Polysiloxanblöcken und aminoorganischen Blöcken, deren Stickstoffatome ggfs. in Form quaternärer Ammoniumgruppen vorliegen, in dem zunächst

So lassen sich reproduzierbar und in der Abfolge der Wiederholeinheiten in der Copolymerkette genau definierte Strukturen herstellen.

Ein Gegenstand der Erfindung ist daher ein Verfahren zur Herstellung von neuartigen linearen Copolymerstrukturen, bestehend aus alternierend abfolgenden Polysiloxanblöcken und aminoorganischen Blöcken, deren Stickstoffatome ggfs. in Form quaternärer Ammoniumgruppen vorliegen, in dem zunächst
a) ein Diglycidylether der Formel (1) Im molaren Überschuss mit mindestens einer Aminverbindung ausgewählt aus Piperazin, N,N'-Dimethylhexamethylen-l,6-diamin und N,N'-Dimethyltetramethylen-1,4-diamin zu einem endständig Glycidylgruppen tragenden kettenverlängerten Addukt umgesetzt wird, wobei das molare Verhältnis von Diepoxld zu Amin variabel ist und vorzugsweise 10:1 bis 1,1:1 beträgt, und
b) dieses Zwischenprodukt anschließend mit einem terminal sek. Aminogruppen tragenden linearen Polysiloxan der Formel (3) vorzugsweise in einem molaren Verhältnis von 1,5:1 bis 0,7:1 und gegebenenfalls in einem Lösemittel zur Reaktion gebracht wird,
c) optional zur vollständigen oder teilweisen Quaternierung der Stickstoffatome an beliebiger Stelle in diesem Verfahren, am günstigsten nach Ende der vorbeschriebenen Umsetzung im noch Lösemittel enthaltenden System, optional Säuren wie vorzugsweise Carbonsäuren, aber auch anorganische Säuren oder Alkylierungsreagentien wie Alkylhalogenlde oder Alkylsulfate zugefügt werden können, und
d) ebenfalls optional Wasser oder organische Verbindungen wie Polyether, Polyole oder Alkohole als Verdünnungsmittel, Emulgatoren oder Modifizierungsmittel zugefügt werden.

Die erfindungsgemäß hergestellten Copolymere sind somit Verbindungen der allgemeinen Formel (4),

H - A - [(B - C)ₓ - B - A]_{y} - H Formel (4)

wobei
- A: ein Siloxanfragment aus der Struktur des Aminosiloxans der Formel (3) ist gemäß Formel (5) 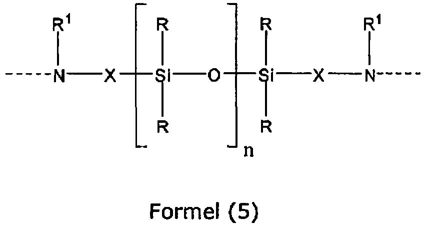
- B: ein organisches Fragment ist, das aus der Epoxidringöffnung des Dlglycldylethers der Formel (1) hervorgeht gemäß Formel (6) 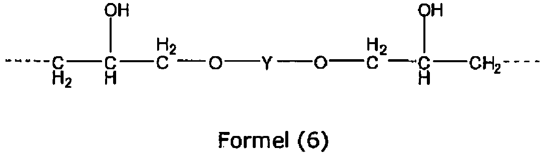
- C: ein Fragment aus der Aminstruktur der dl-sekundären Amine ausgewählt aus Piperazin, N,N'-Dimethylhexamethylen-1,6-diamin und N,N'-Dimethyltetramethylen-1,4-diamin Ist,
und wobei die Reste X, Y, R, R¹, und R² sowie die Indizes nund m die vorgenannten Bedeutungen haben,
- x: eine Zahl von 0,1 bis 10, vorzugsweise 0,2 bis 5 ist,
- y: eine Zahl von 1,1 bis 50, vorzugsweise 1,2 bis 30 ist.

Formel (4) verdeutlicht nicht nur die durch das erfindungsgemäße Herstellverfahren vorgegebene strenge Blockabfolge der Wiederholeinhelten A, B und C, sondern auch die Anbindung des Siloxankörpers an den aminoorganischen Block ausschließlich über eine Amingruppe des Typs -X-N(R¹)-, was die erfindungsgemäßen Copolymere von den nicht (AB)n-Strukturen in der WO 02/092904 klar unterscheidet.

Die in den hier angeführten Formeln wiedergegebenen Indexzahlen und die Wertbereiche der angegebenen Indizes verstehen sich als die Mittelwerte der möglichen statistischen Verteilung der tatsächlich vorhandenen Strukturen und/oder deren Mischungen. Dies gilt auch für als solche an sich exakt wiedergegebene Strukturformeln.

Ein weiterer Gegenstand der Erfindung sind die nach dargestelltem Verfahren herstellbaren Copolymere der Formel (4) und deren mit Säuren und/oder Alkylierungsmitteln quaternisierten, Ammoniumgruppen tragende Derivate.

Ein weiterer Gegenstand der Erfindung sind daher Zusammensetzungen, die die erfindungsgemäßen Copolymere enthalten, insbesondere Konzentrate, Compounds /Emulsionskonzentrate und/oder deren wässerige Formulierungen, wässrige Emulsionen und/oder Lösungen, eine Formulierung oder Emulsion in organischen Verbindungen wie Polyethern, Polyolen, Alkoholen.

Ein weiterer Gegenstand der Erfindung ist die Verwendung der erfindungsgemäßen Verbindungen, erhältlich nach dem genannten Verfahren, als gegebenenfalls permanenter Weichmacher für Flächengebilde ausgewählt beispielsweise aus der Gruppe umfassend Gewebe, textile Gewebe, Gewirke, Gestricke, Nonwovens, Tissue (Papierfaser) und/oder Fasern aus natürlichen und/oder synthetischen Rohstoffen und/oder Leder und/oder Haare und/oder Fell, wobei der Weichmacher den damit behandelten Flächengebilden gegebenenfalls auch hydrophile Eigenschaften verleihen kann. Insbesondere verleihen die erfindungsgemäßen Verbindungen hydrophile Eigenschaften BEI GLEICHZEITIGER Erzielung eines guten Griffs und guter Permanenz.

Es ist eine weitere Aufgabe der Erfindung, Verbindungen und Formulierungen, enthaltend diese Verbindungen zur Verfügung zu stellen, die in der Lage sind, sowohl Eigenschaften wie Kämmbarkeit, Weichheit, Volumen, Formbarkeit, Handhabbarkeit, die Entwirrbarkeit von ungeschädigten und geschädigten Haaren zu verbessern, und/oder auch dem Haar einen schönen Glanz zu verleihen. Die Verbindungen sollen also eine verbesserte oder zumindest gleich gute Einzelwirkung, insgesamt aber eine verbesserte kombinierte Wirkung von mechanischen und anderen Eigenschaften zeigen.

Ein weiterer Gegenstand dieser Erfindung ist die Verwendung der Copolymere der allgemeinen Formel (4) bzw. der diese Verbindungen enthaltenden Mischungen in glanzverbessernden kosmetischen Pflegeformulierungen, als Haarbehandlungsmittel und Haarnachbehandlungsmittel zum Ausspülen oder zum Verbleib im Haar, beispielsweise in Shampoos mit oder ohne ausgeprägte Konditionierwirkung, Konditionierern, 2in1-Shampoos, Spülungen, Haarkuren, Haarmasken, Frisierhilfen, Stylingmittel, Fönlotionen, Haarfestiger, Dauerwellmittel, Haarglättungsmittel und/oder Mitteln zum Färben der Haare.

Ein weiterer Vorteil der erfindungsgemäßen Verwendung ist, dass die Polysiloxane mit quaternären Funktionen gemäß Formel (4) hervorragende konditionierende Effekte auf die Haut ausüben können. Durch diesen konditionierenden Effekt auf der Haut kann einem trockenen, spröden oder rauen Zustand der Haut nach Anwendungen einer kosmetischen wässrigen, tensidischen Formulierung vorgebeugt werden und ein angenehmes, samtigseidiges Hautgefühl erzielt werden.

Ein weiterer Gegenstand der vorliegenden Erfindung sind kosmetische, dermatologische und pharmazeutische Formulierungen sowie Körper-Pflege- und Körper-/Gesichts-Reinigungsmittel, die durch die Verwendung der erfindungsgemäßen Copolymere erhalten werden und z.B. mindestens eine zusätzliche Komponente enthalten, ausgewählt aus der Gruppe umfassend Emollients, Emulgatoren und Tenside, Verdicker/Viskositätsregler/Stabilisatoren, UV-Lichtschutzfilter, Antioxidantien, Hydrotrope (oder Polyole), Fest- und Füllstoffe, Filmbildner, Perlglanzadditive, Deodorant- und Antitranspirantwirkstoffe, Insektrepellentien, Selbstbräuner, Konservierungsstoffe, Konditioniermittel, Parfüme, Farbstoffe, kosmetische Wirkstoffe, Pflegeadditive, Überfettungsmittel, Lösungsmittel. Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Das erfindungsgemäße Verfahren gestattet es durch das gezielte Ansteuern von Blockstrukturen unterschiedlichster Sequenzlänge den Siloxangehalt in Relation zum organischen Anteil und zur Anzahl der ggfs. quaternisierten Stickstoffatome in weiten Grenzen flexibel auf den gewünschten Anwendungsbereich zuzuschneidern. So erzeugen zum Beispiel Copolymere mit einem hohen Siliconanteil einen angenehmen Griff des damit behandelten Gewebes, gleichzeitig haben sie eine relativ niedrige Viskosität, die es erlaubt, derartige Verbindungen wässrig zu formulieren.

Insbesondere verleihen die erfindungsgemäßen linearen organomodifizierten Polysiloxane textilen Geweben einen sehr guten hydrophilen Weichgriff und besitzen eine erhöhte Permanenz auf Textilien. Darüber hinaus sind eine hohe Sprungelastizität und eine verbesserte Entknitterungseigenschaft einer so ausgerüsteten Ware als weitere positive Eigenschaft zu bewerten.

Die erfindungsgemäßen Copolymere können z.B. als Weichmacher für Textilien und Geweben in Form von Konzentraten, Compounds/Emulsionskonzentraten, Formulierungen und daraus hergestellten Flotten verwendet und gegebenenfalls appliziert werden, wobei die Copolymere in solchen Systemen anteilsmäßig zu 0,5 bis 99 Gew.-%, bevorzugt zu 3 bis 70 Gew.-%, im Besonderen zu 5 bis 50 Gew.-% bezogen auf die gesamte Formulierung eingesetzt werden.

Flotte steht für eine meist wässrige Flüssigkeit, in der Textilien gewaschen, gebleicht, gefärbt oder imprägniert werden. Dabei bedeutet der Begriff Flotte die Gesamtheit aus Lösungsmittel (meist Wasser) und allen darin enthaltenen (gelösten, emulgierten oder dispergierten) Bestandteilen wie beispielsweise Farbstoffen, Emulgatoren und weiteren Hilfsmitteln. Die Gesamtheit der in der Flotte gelösten Bestandteile wird gemeinhin auch als Feststoffgehalt bezeichnet, wobei der Feststoffgehalt den Trocknungsrückstand nach Verdampfung der flüchtigen Bestandteile (bei etwa 100 °C - 105 °C) angibt. Die Menge der Komponenten einer Flotte wird meist in g/l bei Flüssigkeiten oder % (bezogen auf das Warengewicht) angegeben.

Im Textilbereich spricht man ganz allgemein von einer Behandlungs-Flotte als dem Bad (meist wässrig), in dem (oder mit dem) das Gewebe mit einem oder mehreren (oberflächenaktiven) Stoffen ausgerüstet wird. Dabei gibt es neben den Hauptsystemen weitere Applikationsformen wie Aufsprühen, Rakeln oder Walzenauftrag, je nach Endanwendung und somit Viskosität des Produktes.

Für wässrige Systeme werden hauptsächlich zwei Systeme verwendet:
Für substantive, also kationische Produkte - Ausziehverfahren ("Exhaustion"), hier wird im Prinzip, wie der Weichspüler in der Waschmaschine, das Gewebe für eine bestimmte Zeit bei bestimmter Temperatur in der Flotte bewegt. Anschließend wird diese abgelassen und das Gewebe getrocknet.
Für nicht-substantive und substantive Produkte - Foulard-Applikation, wie beispielsweise mit einem Labor-Foulard der Firma Matthis (Typ HVF), hier wird das Gewebe durch die Flotte hindurchgeleitet und zwischen den Walzen (im Mangel-Prinzip) auf eine Restfeuchte abgequetscht und anschließend getrocknet.

Weitere Gegenstände der Erfindung sind daher ein Konzentrat, ein Compound/Emulsionskonzentrat, eine Formulierung oder Emulsion gemäß nachfolgender Definitionen.

Als Konzentrat wird die nahezu reine, etwa 90-100 Gew.-%ige Copolymer-Verbindung der Formel (4) bezeichnet, die nur mit geringen Anteilen an Lösungsmitteln versetzt ist - diese sind in der Regel nicht in Wasser löslich und auch nicht selbstemulgierbar.

Compounds oder Emulsionskonzentrate enthalten 50-90 Gew.-%, bevorzugt 50-80 Gew.-% der Copolymeren-Verbindung und als weitere Bestandteile Wasser und/oder Lösungsmittel, ausgewählt aus der Gruppe der Glykole, unverzweigten und/oder verzweigten Alkohole und/oder Alkylether mit 1 bis 6 Kohlenstoffatomen und gegebenenfalls einen oder mehreren nicht-ionischen Emulgatoren, beispielsweise ein Alkoholethoxylat mit 3-25 Ethylenoxideinheiten. Compounds und Emulsionskonzentrate sind in der Regel in Wasser löslich bzw. selbstemulgierbar.

Formulierungen und/oder (wässrige) Emulsionen enthalten 5-20 Gew.-% des erfindungsgemäßen Copolymers, Lösungsmittel, Emulgatoren (auch kationisch oder amphoter), Wasser. Der Feststoffgehalt dieser Formulierungen oder Emulsionen beträgt in der Regel etwa 40 Gew.-%.

Aus den zuvor genannten Konzentraten, Compounds und/oder Formulierungen/Emulsionen werden durch Verdünnen in Wasser in den Herstellbetrieben/Ausrüstbetrieben die (Applikations-)Flotten (Anwendungs-/Ausrüstungsbäder) hergestellt. Typische Flottenkonzentrationen bei der Foulardapplikation sind beispielsweise 5-80 g Formulierung/Emulsion pro Liter Flottenlösung oder Applikationsflotte.

Die erfindungsgemäßen Copolymere können in Haarpflegesystemen in Kombination mit anderen Wirkstoffen und Hilfsmitteln eingesetzt werden. Je nach Anwendungszweck sind solche Mittel enthaltend 2 bis 25 Gew.-% eines oder mehrerer waschaktiver Tenside aus der Gruppe der anionischen, nichtionischen, amphoteren oder zwitterionischen Tenside, 0,5 bis 10 Gew.-% eines oder mehrerer Emulgatoren, 0,5 bis 10 Gew.-% eines oder mehrerer Konsistenzgeber, 0,5 bis 10 Gew.-% eines oder mehrerer vorzugsweise kationischer Tenside oder Emulgatoren, 0,5 bis 20 Gew.-% eines oder mehrerer kosmetischer Öle, Siliconöle oder Emollients sowie übliche Hilfs- und Zusatzstoffe in üblichen Konzentrationen, und zusätzlich enthaltend ein oder mehrere haarkosmetische Wirkstoffe, ausgesucht aus der Gruppe der kationischen Polymere wie zum Beispiel quaternierte Cellulose und dessen Derivate, Chitosan und dessen Derivate, kationische Alkylglycoside, kationische Guar-Derivate, Polymere aus Dimethyldiallylammoniumsalzen und deren Copolymere mit Estern und Amiden von Acrylsäure und Methacrylsäure, Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere, Vinylpyrrolidon-Vinylimidazoliummethochlorid-Copolymere, Terpolymere aus den Monomeren Vinylpyrrolidon, Caprolactam und Acrylamiden, quaternierter Polyvinylalkohol sowie solche Polymere die unter den INCI-Bezeichnungen Polyquaternium-2, Polyquaternium-17, Polyquaternium-18, Polyquaternium-27 und Polyquaternium-37 bekannt sind, kationische oder nichtionische Proteinhydrolysate pflanzlichen oder tierischen Ursprungs auf Basis Keratin, Collagen, Elastin, Weizen, Reis, Soja, Milch, Seide, Mais oder weitere Siliconderivate, wie zum Beispiel Dimethiconol oder Dimethicone (INCI Bezeichnungen für Polydimethylsiloxane) sowie modifizierte Silicone, die terminal funktionalisiert (INCI Vorsilbe Bis-) und/oder pfropf-funktionalisiert sein können, als da sind beispielsweise Alkoxysilicone und Alkylsilicone mit langkettigen Alkylgruppen, polyoxyalkylmodifizierte Silicone wie PEG/PPG-3/10 Dimethicone oder B i s-PEG/PPG-20/20 Dimethicone mit oder ohne Alkylethergruppe und deren Ester, wie zum Beispiel Dimethicone PEG-7 Cocoate sowie multifunktionalisierte Silicone wie zum Beispiel Cetyl PEG/PPG-10/1 Dimethicone oder Methyleugenyl PEG-8 Dimethicone, darüber hinaus Siliconcopolymere mit Acrylaten, einschließlich solcher Copolymere mit und ohne Alkylmodifizierung, verzweigte Siliconderivate wie Dimethicone/Silsesquioxane Copolymer, vernetzte Siliconcopolymere wie DimethiconeCrosspolymer, Alkyl Dimethicone/Divinyldimethicone Crosspolymer, Cetearyl Dimethicone Crosspolymer oder Cetearyl Dimethicone/Vinyl Dimethicone Crosspolymer, aminofunktionalisierte Silicone wie Amodimethicone, Aminopropyl Dimethicone, PEG-7 Amodimethicone, Methoxy PEG/PPG-7/3 Aminopropyl Dimethicone oder ionisch modifizierter Silicone wie Dimethicone Propyl PG-Betaine, Vitamine, Panthenol, Pyrrolidoncarbonsäure, Bisabolol, Pflanzenextrakte, Kreatin, Ceramide sowie UV-absorbierende Mittel.

Weitere Ausgestaltungen und Gegenstände der Erfindung ergeben sich aus den Ansprüchen, deren Offenbarungsgehalt vollumfänglich Gegenstand dieser Beschreibung ist.

Die erfindungsgemäßen Copolymere mit Amino- und/oder quaternären Ammoniumgruppen und das Verfahren zu deren Herstellung werden nachfolgend beispielhaft beschrieben, ohne dass die Erfindung auf diese beispielhaften Ausführungsformen beschränkt sein soll.

Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können.

### Experimenteller Teil:

Für die erfindungsgemäße Herstellung der Copolymere wurden die folgenden linearen Aminosiloxane eingesetzt, die nach dem in US 5,486,634 beschriebenen Verfahren durch Hydrosilylierung der entsprechenden α,ω-SiH-Polydimethylsiloxane mit N-Ethylmethylallylamin gewonnen wurden:

| Aminosiloxan 1 | Aminosiloxan 2 | Aminosiloxan 3 |
|---|---|---|
| Mittlere Molmasse 6100 g/mol | Mittlere Molmasse 3900 g/mol | Mittlere Molmasse 2400 g/mol |
| ca. 80 Dimethylsiloxyeinheiten in der Polymerkette | ca. 50 Dimethylsiloxyeinheiten in der Polymerkette | ca. 30 Dimethylsiloxyeinheiten in der Polymerkette |

Als Diepoxid wurde ein Polypropylenglykoldiglycidylether der Firma DOW Epoxy Systems (Polypox^{®} R19) mit einem Epoxidäquivalentgewicht von 329 g/mol und einer Epoxidzahl von 171 mg KOH/g eingesetzt.

Die Viskositäten wurden in Anlehnung an die DIN 53019 mit einem Rotationsviskosimeter der Marke Brookfield (Model LVT) bei 25°C gemessen.

### Bestimmung des Siloxanquat-Stickstoffgehaltes:

Die Bestimmung des quaternären Stickstoffs erfolgt mittels potentiometrischer Titration mit einer Dodecylsulfat-Lösung als Titrant unter Verwendung einer Elektrodenkombination bestehend aus einer speziellen, lösemittelbeständigen Sensorelektrode, z.B. der Surfactrode Resistant (Metrohm AG) und einer Bezugselektrode (Ag/AgCl- Kartusche double junction, Metrohm AG). Die auf Quat-Stickstoff zu bestimmende Probe wird mit einer Genauigkeit von 0.1 mg in einen Titrationsbecher eingewogen. Nach Auflösen in 10 ml MIBK pipettiert man 10 ml vergällten Ethanol und 0,2 mL TEGO^{®} add (Fa. Metrohm AG Art. Nr. 6.2317.100) hinzu, fügt dann 10 mL Pufferlösung pH 10 hinzu und verdünnt mit 80 ml dest. Wasser. Anschließend wird am Titroprozessor gegen eine 0.005 molare Natriumdodecylsulfat-Lösung titriert. Unter Berücksichtigung des Verbrauchs dieser Titerlösung und der Einwaage wird der Gehalt an quaternärem Stickstoff berechnet.

### Herstellung der Diepoxid-Diamin-Adddukte:

### Addukt 1:

In einem Glaskolben werden 400 g des Diepoxids Polypox^{®} R19 vorgelegt und unter Stickstoffinertisierung auf 80 °C erwärmt. Innerhalb von 1 h werden unter Rühren und Kühlen portionsweise 25,6 g eines bei ca. 50 °C aufgeschmolzenen Piperazins (68 %ig in Wasser) zugefügt. Nach 2 h Nachreaktion entsteht ein gelbliches, klares Reaktionsprodukt (Viskosität bei 25 °C: 1020 mPas).

### Addukt 2:

In einem Glaskolben werden 400 g des Diepoxids Polypoc^{®} R19 vorgelegt und unter Stickstoffinertisierung auf 80 °C erwärmt. Innerhalb von 1 h werden unter Rühren und Kühlen portionsweise 30,4 g eines bei ca. 50 °C aufgeschmolzenen Piperazins (68 %ig in Wasser) zugefügt. Nach 2 h Nachreaktion entsteht ein gelbliches, klares Reaktionsprodukt (Viskosität bei 25 °C: 1100 mPas).

### Addukt 3:

In einem Glaskolben werden 400 g des Diepoxids Polypox^{®} R19 vorgelegt und unter Stickstoffinertisierung auf 80 °C erwärmt. Innerhalb von 1 h werden unter Rühren und Kühlen portionsweise 38,0 g eines bei ca. 50 °C aufgeschmolzenen Piperazins (68 %ig in Wasser) zugefügt. Nach 2 h Nachreaktion entsteht ein gelbliches, klares Reaktionsprodukt (Viskosität bei 25 °C: 2870 mPas).

### Herstellung der erfindungsgemäßen Copolymere:

### Copolymer 1:

2500 g Aminosiloxan 1 und 2500 g Isopropanol werden in einem mit Rückflusskühler ausgestatteten Glaskolben vorgelegt und unter Stickstoffbeschleierung auf 80 °C erwärmt. Innerhalb von 45 min werden 383,8 g Addukt 1 unter Rühren zugegeben. Nach 2 h Nachreaktion bei 80-82 °C (Rückfluss) wird der Rückflusskühler gegen eine Destillationsbrücke getauscht und Isopropanol wird bei Sumpftemperaturen bis 110 °C und Vakua von minimal 20 mbar destillativ entfernt. Nach Entspannen mit Stickstoff und Abkühlung auf <80 °C werden unter Rühren 16,7 g Essigsäure zugefügt. Nach 20 min Rührzeit wird das klare gelbliche Produkt abgelassen (Viskosität bei 25 °C: 8500 mPas). Der titrimetrisch bestimmte Siloxanquat-Stickstoffgehalt entspricht der Theorie.

### Copolymer 2:

250 g Aminosiloxan 2 und 250 g Isopropanol werden in einem mit Rückflusskühler ausgestatteten Glaskolben vorgelegt und unter Stickstoffinertisierung auf 80 °C erwärmt. Innerhalb von 45 min werden 67,2 g Addukt 2 unter Rühren zugegeben. Nach 2 h Nachreaktion bei 80-82 °C (Rückfluss) wird der Rückflusskühler gegen eine Destillationsbrücke getauscht und Isopropanol wird bei Sumpftemperaturen bis 110 °C und Vakua von minimal 20 mbar destillativ entfernt. Nach Entspannen mit Stickstoff und Abkühlung auf <80 °C werden unter Rühren 1,8 g Essigsäure zugefügt. Nach 20 min Rührzeit wird das leicht trübe gelbliche Produkt abgelassen (Viskosität bei 25 °C: 5150 mPas). Der titrimetrisch bestimmte Siloxanquat-Stickstoffgehalt entspricht der Theorie.

### Copolymer 3:

250 g Aminosiloxan 1 und 250 g Isopropanol werden in einem mit Rückflusskühler ausgestatteten Glaskolben vorgelegt und unter Schutzgasinertisierung auf 80 °C erwärmt. Innerhalb von 45 min unter Rühren werden 52,2 g Addukt 3 zugegeben. Nach 2 h Nachreaktion bei 80-82 °C (Rückfluss) wird der Rückflusskühler gegen eine Destillationsbrücke getauscht und Isopropanol wird bei Sumpftemperaturen bis 110 °C und Vakua von minimal 20 mbar destillativ entfernt. Nach Entspannen mit Stickstoff und Abkühlung auf <80 °C werden unter Rühren 1,8 g Essigsäure zugefügt. Nach 20 min Rührzeit wird das leicht trübe gelbliche Produkt abgelassen (Viskosität bei 25 °C: ca. 10500 mPas). Der titrimetrisch bestimmte Siloxanquat-Stickstoffgehalt entspricht der Theorie.

### Copolymer 4:

250 g Aminosiloxan 3 und 250 g Isopropanol werden in einem mit Rückflusskühler ausgestatteten Glaskolben vorgelegt und unter Stickstoffinertisierung auf 80 °C erwärmt. Innerhalb von 45 min werden 97,5 g Addukt 1 unter Rühren zugegeben. Nach 2 h Nachreaktion bei 80-82 °C (Rückfluss) wird der Rückflusskühler gegen eine Destillationsbrücke getauscht und Isopropanol wird bei Sumpftemperaturen bis 110 °C und Vakua von minimal 20 mbar destillativ entfernt. Nach Entspannen mit Stickstoff und Abkühlung auf <80 °C werden unter Rühren 2,3 g Essigsäure zugefügt. Nach 20 min Rührzeit wird das klare gelbliche Produkt abgelassen (Viskosität bei 25 °C: 4200 mPas). Der titrimetrisch bestimmte Siloxanquat-Stickstoffgehalt entspricht der Theorie.

### Copolymer 5:

150 g Aminosiloxan 1 und 150 g Isopropanol werden in einem mit Rückflusskühler ausgestatteten Glaskolben vorgelegt und unter Stickstoffbeschleierung auf 80 °C erwärmt. Innerhalb von 10 min werden 23,0 g Addukt 1 unter Rühren zugegeben. Nach 2,45 h Nachreaktion bei 80-82 °C (Rückfluss) wird der Rückflusskühler gegen eine Destillationsbrücke getauscht und Isopropanol wird bei Sumpftemperaturen bis 110 °C und Vakua von minimal 20 mbar destillativ entfernt. Nach Entspannen mit Stickstoff und Abkühlung auf <80 °C werden unter Rühren 1,6 g Essigsäure zugefügt. Nach 20 min Rührzeit wird das klare gelbliche Produkt abgelassen (Viskosität bei 25 °C: 9000 mPas). Der titrimetrisch bestimmte Siloxanquat-Stickstoffgehalt entspricht der Theorie.

### Copolymer 6:

150 g Aminosiloxan 1 und 150 g Isopropanol werden in einem mit Rückflusskühler ausgestatteten Glaskolben vorgelegt und unter Stickstoffbeschleierung auf 80 °C erwärmt. Innerhalb von 15 min werden 25,0 g Addukt 1 unter Rühren zugegeben. Nach 2 h Nachreaktion bei 80-82 °C (Rückfluss) wird der Rückflusskühler gegen eine Destillationsbrücke getauscht und Isopropanol wird bei Sumpftemperaturen bis 110 °C und Vakua von minimal 20 mbar destillativ entfernt. Nach Entspannen mit Stickstoff und Abkühlung auf <80 °C werden unter Rühren 1,0 g Essigsäure zugefügt. Nach 20 min Rührzeit wird das gelbliche opake Produkt abgelassen. Der titrimetrisch bestimmte Siloxanquat-Stickstoffgehalt entspricht der Theorie.

### Anwendungsbeispiele:

### Allgemeine Formulierung:

5-50 Gew.-% des Aminosiloxans werden in einem Becherglas mit Propellerrührer unter Rühren vorgelegt. Anschließend werden der Reihe nach 5-25 Gew.-% Dipropylenglykol bzw. Butyldiglycol, 3-15 Gew.-% eines Fettalkoholethoxylates mit einem Ethoxylierungsgrad von 6 unter Rühren hinzugegeben. Zuletzt wird mit Wasser auf 100 Gew.-% aufgefüllt.

### Formulierung 1 - erfindungsgemäß:

20 Teile des erfindungsgemäßen Copolymers 1 mit quaternären Ammoniumgruppen aus Beispiel 1 werden in einem Becherglas mit Propellerrührer unter Rühren vorgelegt. Anschließend werden der Reihe nach 10 Teile Dipropylenglykol, 10 Teile eines Fettalkoholethoxylates mit einem Ethoxylierungsgrad von 6 unter Rühren hinzugegeben. Zuletzt wird mit 60 Teilen Wasser aufgefüllt. Man erhält klare niedrigviskose Formulierung.

### Formulierung 2 - erfindungsgemäß:

Analog zur Herstellung von Formulierung 1 wurde aus dem erfindungsgemäßen Copolymer 5 die Formulierung 2 hergestellt.

### Formulierung 3 - nicht erfindungsgemäß:

Eine Emulsion gemäß Formulierung 1 wurde mit einem handelsüblichen Siliconquat, Tegopren® 6924, hergestellt. Bei Tegopren^{®} 6924 handelt es sich um ein lineares Siloxan mit endständiger Modifizierung durch Quatfunktionen.

### Formulierung 4 - nicht erfindungsgemäß:

Eine Emulsion eines handelsüblichen Aminosiloxans (z. B. Biosoft von BioTex) wurde mit einem Aktivgehalt von 20 Gew.-% hergestellt.

### Formulierung 5 - erfindungsgemäß:

Analog zur Herstellung von Formulierung 1 wurde aus dem erfindungsgemäßen Copolymer 6 die Formulierung 5 hergestellt.

### Formulierung 6 - nicht erfindungsgemäß:

Tegopren^{®} 7100, eine handelsübliche Emulsion eines seitenständig modifizierten Siloxans, wobei als Modifizierung Polyether- und Aminofunktionen nebeneinander auftreten wurde mit einem Aktivgehalt von 20 Gew.-% hergestellt.

### Applikationsbeispiele:

Zur Überprüfung des bei Anwendung der Aminosiloxane erzielbaren Griffes (haptische Bewertung) sowie auch der erreichbaren Hydrophilie wurden aus nativen Fasern bestehende Produkte gemäß folgendem Verfahren hiermit ausgerüstet:

### Foulardverfahren:

Zur Ausprüfung des Weichgriffs der jeweiligen Emulsionen wurden Baumwollwirkware (160 g/m²) und Baumwoll-Frottierware (400 g/m²) mit einer Flotte, die jeweils 20 g/l der entsprechenden Emulsion enthielt, foulardiert, danach wurden die Textilien bis auf ca. 100 Gew.-% Flottenaufnahme abgequetscht und drei Minuten bei einer Temperatur von 130 °C getrocknet.

Zur Ausprüfung der Hydrophilie wurde Baumwollwebware (200 g/m²) mit einer Flotte, die jeweils 30 g/l der entsprechenden Emulsion enthielt, foulardiert und dann auf ca. 100 Gew.-% Flottenaufnahme abgequetscht und drei Minuten lang bei 130 °C getrocknet.

### Testmethoden:

### Griffbeurteilung:

Zur Beurteilung des Warengriffes wurde ein erfahrenes Team zusammengestellt, das die anonymisierten Griffmuster, der mit den Emulsionen ausgerüsteten Wirk- und Frottierwaren, mit Hilfe eines Handpaneltests bewertete. Bei den Griffmustern aus Maschenware wurde zusätzlich eine nicht offensichtlich gekennzeichnete unbehandelte Probe hinzugelegt.

### Prüfung der Hydrophilie:

Zur Überprüfung der Hydrophilie wurde die an DIN 53924 angelehnte Prüfmethode zur Messung der Steighöhe von Wasser verwendet. Dabei wird das ausgerüstete Baumwolltestgewebe in jeweils fünf 25 cm lange und 1,5 cm breite Streifen geschnitten, mit einem wasserlöslichen Stift seitlich markiert und an einer Halterung senkrecht straff aber ohne Spannung befestigt. Die Halterung wird anschließend für fünf Minuten so in ein Wasserbecken gestellt, dass 2 cm der Streifen ins Wasser eintauchen. Die wasserlösliche Markierung dient der besseren Erkennbarkeit der Steighöhe durch das Verlaufen der Farbe bei Benetzung mit Wasser. Nachdem die Halterung 10 Minuten außerhalb des Wasserbeckens gestanden hat, wird die Steighöhe in cm abgelesen und gegen den Blindwerte (Steighöhe der unbehandelten Baumwollstreifen x cm = 100%) bestimmt und in % vom Blindwert angegeben.

### Waschvorgang:

Die Waschvorgänge wurden in einer handelsüblichen Waschmaschine, Miele Novotronic W 918 mit Buntwäsche ohne Vorwaschen bei 40 °C mit wfk Standardwaschmittel IECA-Base und 3 kg B W-Ballastgewebe gewaschen. Zuletzt wurde das so behandelte Gewebe 12 Stunden lang bei Raumtemperatur getrocknet.

Die Testergebnisse bezüglich des Weichgriffs sind in den Tabellen 1 bis 3 und bezüglich der Hydrophilie in der Tabelle 4 aufgeführt.

**Tabelle 1:**

| Weichgriffbeurteilung auf Baumwollwirkware nach Applikation durch Foulard | |
|---|---|
| Formulierung 1 Erfindungsgemäß | +++ |
| Formulierung 2 Erfindungsgemäß | ++ |
| Formulierung 3 nicht erfindungsgemäß | +++ |
| Formulierung 4 nicht erfindungsgemäß | +++ |
| Unbehandelt | - |

| | |
|---|---|
| +++ exzellent, ++ sehr gut, + gut, o befriedigend, - schlecht | |

**Tabelle 2:**

| Weichgriffbeurteilung auf Baumwollfrottierware nach Applikation durch Foulard | |
|---|---|
| Formulierung 1 Erfindungsgemäß | ++ |
| Formulierung 2 Erfindungsgemäß | +++ |
| Formulierung 3 nicht erfindungsgemäß | ++ |
| Formulierung 4 nicht erfindungsgemäß | ++ |
| Unbehandelt | - |

| | |
|---|---|
| +++ exzellent, ++ sehr gut, + gut, o befriedigend, - schlecht | |

**Tabelle 3:**

| Weichgriffbeurteilung auf Baumwollwirkware nach Applikation durch Foulard | | | | |
|---|---|---|---|---|
| | Vor der Wäsche | Nach der 1. Wäsche | Nach der 3. Wäsche | Nach der 5. Wäsche |
| Formulierung 3 nicht erfindungsgemäß | ++ | ++ | ○ | ○ |
| Formulierung 4 nicht erfindungsgemäß | +++ | +++ | +++ | ++ |
| Formulierung 5 Erfindungsgemäß | +++ | +++ | ++ | + |
| Formulierung 6 nicht erfindungsgemäß | + | ○ | - | - |
| Unbehandelt | ○ | - | - | - |

| | | | | |
|---|---|---|---|---|
| +++ exzellent, ++ sehr gut, + gut, o befriedigend, - schlecht | | | | |

**Tabelle 4:**

| Beurteilung der Wasseraufnahme von Baumwollwebware nach Applikation durch Foulard | |
|---|---|
| Gewebe Typ der behandelten Baumwolle | Steighöhe in % des Blindwertes |
| Formulierung 1 - erfindungsgemäß | 84,3 |
| Formulierung 2 - erfindungsgemäß | 82,6 |
| Formulierung 3 - nicht erfindungsgemäß | 84,1 |
| Formulierung 4 - nicht erfindungsgemäß | 30,3 |
| Formulierung 5 - erfindungsgemäß | 81,9 |
| Formulierung 6 - nicht erfindungsgemäß | 90,5 |
| Unbehandelt | 100,0 |

Die Ergebnisse der Tabelle 4 zeigen eine exzellente Wasseraufnahme der mit den erfindungsgemäßen Formulierungen behandelten Baumwollwebwaren von über 80% der Steighöhe der unbehandelten Probe. Damit ist die erhöhte Hydrophilie der erfindungsgemäßen Formulierungen, insbesondere gegenüber der nicht erfindungsgemäßen Formulierung 4 belegt. Gleichzeitig zeigen die mit den erfindungsgemäßen Formulierungen behandelten textilen Flächengebilde einen exzellenten Weichgriff verbunden mit einer sehr guten Permanenz (Formulierung 5 in Tabelle 3). Ein exzellenter Weichgriff verbunden mit guter Permanenz kann zwar auch mit der nicht erfindungsgemäßen Formulierung 4 erreicht werden, diese zeigt jedoch ein außerordentlich hydrophobes und damit unerwünschtes Verhalten bezüglich der Wasseraufnahme.

Tabelle 4 zeigt weiterhin, dass die Ergebnisse der nicht erfindungsgemäßen Formulierungen 3 und insbesondere 6 bezüglich der Wasseraufnahme zwar ähnlich zu den erfindungsgemäßen sind, allerdings ist bei diesen Formulierungen der Weichgriff auf Baumwollwirkware wesentlich schlechter, wie es der Tabelle 3 zu entnehmen ist.

### Erläuterung:

Es resultiert ein weicher, sehr flauschiger und seidiger Griff der mit den erfindungsgemäßen Produkten ausgerüsteten textilen Flächengebilde (Formulierungen 1, 2 und 5), der auch nach mehrmaligem Waschen im Wesentlichen erhalten bleibt (Formulierung 5). Die erfindungsgemäßen Produkte zeigen einen exzellenten Griff bei gleichzeitig exzellenter Hydrophilie im Vergleich zu handelsüblichen Produkten.

Formulierung 5 zeigt zudem auf glattem Gewebe (Baumwollwirkware) eine über fünf Wäschen deutlich verbesserte Permanenz, welche sich in einer annähernd gleichbleibend guten Griffbewertung widerspiegelt.

## Patentansprüche

1. Lineare Polydimethylsiloxan-Polyether-Copolymere mit Amino- und/oder quaternären Ammoniumgruppen der Formel (4)
H-A-[(B-C)ₓ-B-A]_{y}-H Formel (4)
wobei
A ein Siloxanfragment aus der Struktur des Aminosiloxans der Formel (3) ist gemäß Formel (5) 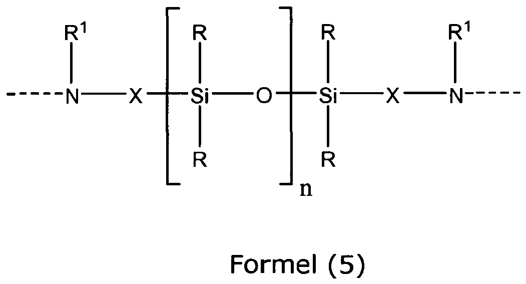 wobei
R eine Methylgruppe darstellt,
R¹ ein beliebiger Kohlenwasserstoffrest ist,
X ein divalenter linearer oder verzweigter Kohlenwasserstoffrest mit 1-20 C-Atomen ist, welcher durch Stickstoffatome bzw. aminische Gruppen unterbrochen sein kann, und
n eine ganze Zahl von 1 bis 500 darstellt;
B ein organisches Fragment ist, dass aus der Epoxidringöffnung des Diglycidylethers der Formel (1) hervorgeht gemäß Formel (6) 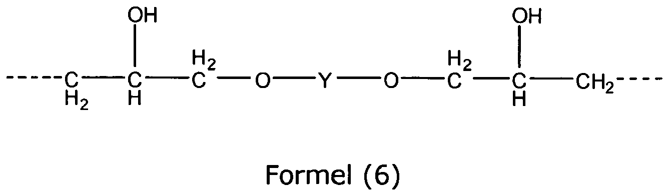
wobei
Y ein divalenter Polyetherrest des Typs - (CH₂ - CHR² - O)ₘ- CH₂-CHR² - und m eine ganze Zahl von 0 bis 50 und R² Wasserstoff oder eine Alkylgruppe mit 1-4 C-Atomen ist;
C ein Fragment aus der Aminstruktur der di-sekundären Amine ausgewählt aus Piperazin, N,N'-Dimethylhexamethylen-1,6-diamin und N,N'-Dimethyltetramethylen-1,4-diamin ist,
x eine Zahl von 0,1 bis 10 ist,
y eine Zahl von 1,1 bis 50 ist
erhältlich durch die Umsetzung von mit sekundären Aminoalkylgruppen funktionalisierten Organopolysiloxanen mit den Reaktionsprodukten aus Epoxidgruppen enthaltenden Verbindungen und Aminen.

2. Verfahren zur Herstellung von linearen Polydimethylsiloxan-Polyether-Copolymeren mit Amino- und/oder quaternären Ammoniumgruppen, **dadurch gekennzeichnet, dass** zunächst
a) ein Diepoxid mit mindestens einer Aminverbindung zu einem endständig Glycidylgruppen tragenden kettenverlängerten Addukt umgesetzt wird, wobei das molare Verhältnis von Diepoxid zu Amin variabel aber mindestens größer 1 zu 1 ist, und
b) dieses Zwischenprodukt anschließend mit einem terminal sek. Aminogruppen tragenden linearen Polysiloxan zur Reaktion gebracht wird,
c) optional zur vollständigen oder teilweisen Quaternierung der Stickstoffatome an beliebiger Stelle in diesem Verfahren Säuren oder Alkylierungsreagentien zugefügt werden können, und
d) weiterhin optional Wasser oder organische Verbindungen ausgewählt aus der Gruppe der Polyether, Polyole oder Alkohole als Verdünnungsmittel, Emulgatoren oder Modifizierungsmittel zugefügt werden.

3. Verfahren nach Anspruch 2, **dadurch gekennzeichnet, dass** als Diepoxide Verbindungen der Formel (1) verwendet werden 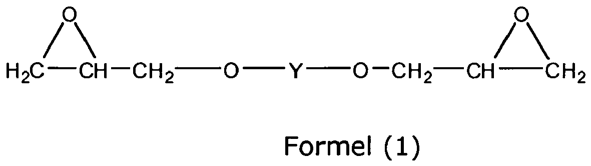 wobei
Y ein divalenter Polyetherrest des Typs - (CH₂ - CHR² - O)ₘ- CH₂-CHR² - und m eine ganze Zahl von 0 bis 50 und R² Wasserstoff oder eine Alkylgruppe mit 1-4 C-Atomen ist..

4. Verfahren nach zumindest einem der Ansprüche 2 oder 3, **dadurch gekennzeichnet, dass** als Aminverbindungen des Verfahrensschritts (a) di-sekundäre Amine ausgewählt aus Piperazin und/oder N,N'-Dimethylhexamethylen-1,6-diamin und/oder N,N'-Dimethyltetramethylen-1,4-diamin verwendet werden.

5. Verfahren nach zumindest einem der Ansprüche 2 bis 4, **dadurch gekennzeichnet, dass** als Verbindungen des Reaktionsschritts (b) lineare, terminal sekundäre Amingruppen aufweisende Polysiloxane gemäß Formel (3) verwendet werden, 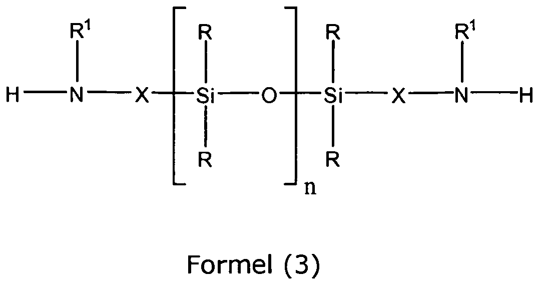 wobei
R¹ R eine Methylgruppe darstellt, ein beliebiger Kohlenwasserstoffrest ist,
X ein divalenter linearer oder verzweigter Kohlenwasserstoffrest mit 1-20 C-Atomen ist, welcher durch Stickstoffatome bzw. aminische Gruppen unterbrochen sein kann, und
n eine ganze Zahl von 1 bis 500 darstellt.

6. Verfahren zur Herstellung von Verbindungen nach zumindest einem der Ansprüche 2 bis 5 enthaltend quaternäre Ammoniumgruppen, **dadurch gekennzeichnet, dass** im Verfahrensschritt (c) eine Säure oder ein Alkylierungsreagenz zugesetzt wird.

7. Copolymere der Formel (4) nach Anspruch 1, und deren mit Säuren und/oder Alkylierungsmitteln quaternisierten, Ammoniumgruppen tragende Derivate.

8. Zusammensetzungen enthaltend Copolymere gemäß der Ansprüche 1 oder 7, diese enthaltende Konzentrate, Compounds /Emulsionskonzentrate und/oder deren wässerige Formulierungen, wässrige Emulsionen und/oder Lösungen, eine Formulierung oder Emulsion in organischen Verbindungen wie Polyethern, Polyolen, Alkoholen.

9. Verwendung der der Copolymere gemäß der Ansprüche 1, sowie 7 bis 8, erhältlich nach zumindest einem der Verfahren der Ansprüche 2 bis 6 als permanente Weichmacher für Flächengebilde ausgewählt aus der Gruppe umfassend Gewebe, textile Gewebe, Gewirke, Gestricke, Non-wovens, Tissue, Papierfaser und/oder Fasern aus natürlichen und/oder synthetischen Rohstoffen und/oder Leder und/oder Haare und/oder Fell.

10. Verwendung nach Anspruch 9, **dadurch gekennzeichnet, dass** der Weichmacher den damit behandelten Flächengebilden hydrophile Eigenschaften verleiht.

11. Verwendung nach zumindest einem der Ansprüche 9 oder 10 in Formulierungen, **dadurch gekennzeichnet, dass** sich die Kämmbarkeit, Weichheit, das Volumen, die Formbarkeit, Handhabbarkeit, die Entwirrbarkeit von ungeschädigten und geschädigten Haaren verbessert, und/oder dem Haar Glanz verliehen wird.

12. Verwendung gemäß zumindest einem der Ansprüche 9 bis 11 in, die Copolymere der allgemeinen Formel (4) oder die Zusammensetzungen enthaltenden Mischungen in, glanzverbessernden kosmetischen Pflegeformulierungen, als Haarbehandlungsmittel und Haarnachbehandlungsmittel zum Ausspülen oder zum Verbleib im Haar, Konditionierern, 2in1-Shampoos, Spülungen, Haarkuren, Haarmasken, Frisierhilfen, Stylingmittel, Fönlotionen, Haarfestiger, Dauerwellmittel, Haarglättungsmittel und/oder Mitteln zum Färben der Haare.

13. Verwendung gemäß zumindest einem der Ansprüche 9 bis 10 in, die Copolymere der allgemeinen Formel (4) oder die Zusammensetzungen enthaltenden Mischungen für kosmetische, dermatologische und pharmazeutische Formulierungen sowie kosmetischen Pflege- und Körper-/Gesichts-Reinigungsmitteln, enthaltend mindestens eine zusätzliche Komponente ausgewählt aus der Gruppe umfassend Emollients, Emulgatoren und Tenside, Verdicker/Viskositätsregler/Stabilisatoren, UV-Lichtschutzfilter, Antioxidantien, Hydrotrope oder Polyole, Fest- und Füllstoffe, Filmbildner, Perlglanzadditive, Deodorant- und Antitranspirantwirkstoffe, Insektrepellentien, Selbstbräuner, Konservierungsstoffe, Konditioniermittel, Parfüme, Farbstoffe, kosmetische Wirkstoffe, Pflegeadditive, Überfettungsmittel, Lösungsmittel.

14. Verwendung gemäß zumindest einem der Ansprüche 9 bis 10, sowie 13 in, die Copolymere der allgemeinen Formel (4) oder die Zusammensetzungen enthaltenden Mischungen für kosmetischen Anwendungen in Form wässriger, tensidischer Formulierung zur Erzielung eines angenehmen, samtig-seidigen Hautgefühls.

## Claims

1. Linear polydimethylsiloxane-polyether copolymers with amino and/or quaternary ammonium groups of the formula (4)
H - A - [(B - C)ₓ - B - A] y - H Formula (4)
where
A is a siloxane fragment from the structure of the aminosiloxane of the formula (3) according to formula (5) 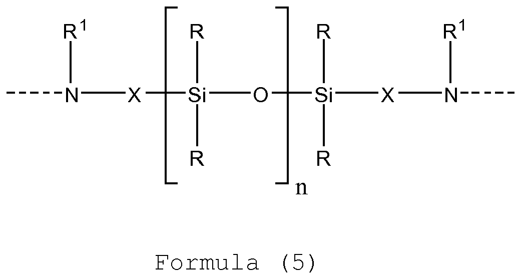 where
R is methyl group,
R¹ is any hydrocarbon radical,
X is a divalent linear or branched hydrocarbon radical which has 1-20 carbon atoms and may be interrupted by nitrogen atoms or aminic groups, and
n is an integer from 1 to 500
B is an organic fragment which arises from the epoxy ring opening of the diglycidyl ether of the formula (1) according to formula (6) 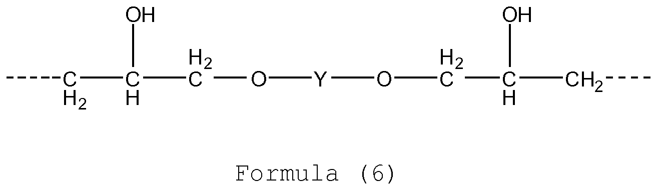 where
Y is a divalent polyether radical of the -(CH₂-CHR²-O)ₘ-CH₂-CHR²- type, and m is an integer from 0 to 50 and R² is hydrogen or an alkyl group having 1-4 carbon atoms;
C is a fragment from the amine structure of the di-secondary amines selected from piperazine, N,N'-dimethylexamethylene-1,6- diamine and N,N'-dimethyltetramthylene-1,4- diamine,
x is from 0.1 to 10,
y is from 1.1 to 50,
obtainable by the reaction of organopolysiloxanes functionalized with secondary aminoalkyl groups with the reaction products formed from compounds containing epoxy groups and amines.

2. Process for preparing linear polydimethylsiloxane-polyether copolymers with amino and/or quaternary ammonium groups, **characterized in that**
a) a diepoxide is first reacted with at least one amine compound to give a chain-extended adduct bearing a terminal glycidyl group, the molar ratio of diepoxide to amine being variable but at least greater than 1 : 1, and
b) this intermediate is then reacted with a linear polysiloxane bearing a terminal secondary amino group,
c) optionally, the nitrogen atoms can be completely or partially quaternized at any point in this process by adding acids or alkylating reagents, and
d) also optionally, water or organic compounds selected from the group of the polyethers, polyols or alcohols can be added as diluents, emulsifiers or modifiers.

3. Process according to Claim 2, **characterized in that** the diepoxides used are compounds of the formula (1) 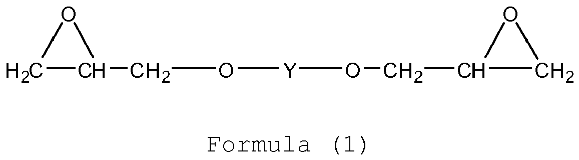 where
Y is a divalent polyether radical of the - (CH₂ - CHR² - O)ₘ- CH₂ -CHR² - type, where m is an integer from 0 to 50 and R² is hydrogen or an alkyl group having 1-4 carbon atoms.

4. Process according to at least either one of Claims 2 and 3, **characterized in that** the amine compounds used in process step (a) are di-secondary amines selected from piperazine and/or N,N'-dimethylhexamethylene-1,6-diamine and/or N,N'-dimethyltetramethylene-1,4-diamine.

5. Process according to at least one of Claims 2 to 4, **characterized in that** the compounds used in reaction step (b) are linear polysiloxanes which have terminal secondary amino groups and are of the formula (3) 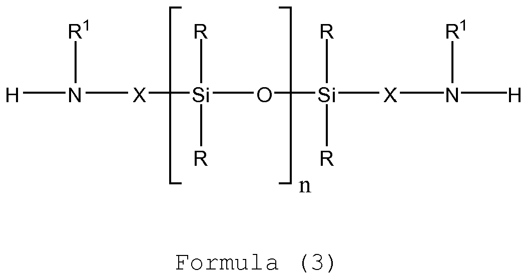 where
R is methyl group,
R¹ is any hydrocarbon radical,
X is a divalent linear or branched hydrocarbon radical which has 1-20 carbon atoms and may be interrupted by nitrogen atoms or aminic groups, and
n is an integer from 1 to 500.

6. Process for preparing compounds according to at least one of Claims 2 to 5, comprising quaternary ammonium groups, **characterized in that** an acid or an alkylating reagent is added in process step (c).

7. Copolymers of the formula (4) according to Claim 1, and the derivatives thereof which have been quaternized with acids and/or alkylating agents and bear ammonium groups.

8. Compositions comprising copolymers according to Claims 1 or 7, concentrates, compounds/emulsion concentrates and/or aqueous formulations, aqueous emulsions and/or solutions thereof, comprising said copolymers, a formulation or emulsion in organic compounds such as polyethers, polyols, alcohols.

9. Use of the copolymers according to Claims 1 and 7 to 8, obtainable by at least one process of Claims 2 to 6, as permanent softeners for fabrics selected from the group comprising wovens, textile wovens, knits, nonwovens, tissue, paper fibre and/or fibres of natural and/or synthetic raw materials and/or leather and/or hair and/or hide.

10. Use according to Claim 9, **characterized in that** the softener imparts hydrophilic properties to the fabrics treated therewith.

11. Use according to at least one of Claims 9 and 10 in formulations, **characterized in that** it improves combability, softness, volume, shapeability, manageability and disentangleability of undamaged and damaged hair, and/or imparts shine to the hair.

12. Use according to at least one of Claims 9 to 11 in mixtures comprising the copolymers of the general formula (4) or the compositions in shine-improving care formulations, as hair treatment compositions and hair aftertreatment compositions to be rinsed out of or to remain in the hair, conditioners, 2 in 1 shampoos, rinses, hair repair treatments, hair masks, styling aids, styling compositions, hair drying lotions, hair-setting compositions, permanent wave compositions, hair smoothing compositions and/or compositions for dyeing the hair.

13. Use according to at least one of Claims 9 and 10 in mixtures comprising the copolymers of the general formula (4) or the compositions for cosmetic, dermatological and pharmaceutical formulations, and cosmetic care and body/face cleansing compositions, comprising at least one additional component selected from the group comprising emollients, emulsifiers and surfactants, thickeners/viscosity regulators/stabilizers, UV light protection filters, antioxidants, hydrotropes or polyols, solids and fillers, film formers, pearlescent additives, active deodorant and antiperspirant ingredients, insect repellents, self-tanning agents, preservatives, conditioners, perfumes, dyes, active cosmetic ingredients, care additives, superfatting agents, solvents.

14. Use according to at least one of Claims 9 to 10 and 13 in mixtures comprising the copolymers of the general formula (4) or the compositions for cosmetic applications in the form of an aqueous surfactant formulation for achieving a pleasant, silky-smooth skinfeel.

## Revendications

1. Copolymères linéaires de polydiméthylsiloxane-polyéther présentant des groupes amino et/ou d'ammonium quaternaire de formule (4)
H-A- [(B-C)ₓ-B-A]-H Formule (4)
où
A représente un fragment siloxane de la structure de l'aminosiloxane de formule (3) selon la formule (5) 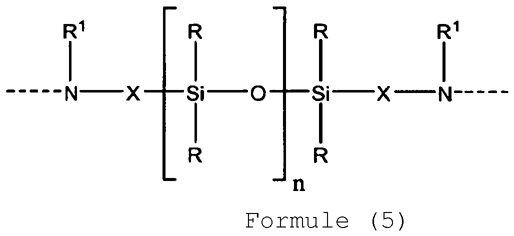 où
R représente un groupe méthyle,
R¹ représente un quelconque radical hydrocarboné,
X représente un radical hydrocarboné divalent, linéaire ou ramifié, comprenant 1-20 atomes de carbone, qui peut être interrompu par des atomes d'azote ou des groupes aminiques, et
n représente un nombre entier de 1 à 500 ;
B représente un fragment organique qui découle de l'ouverture de cycle époxyde du diglycidyléther de formule (1) selon la formule (6) 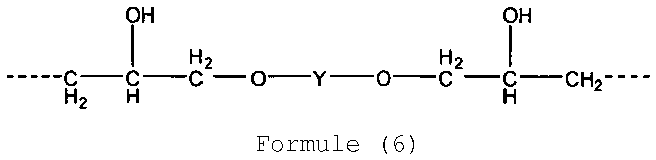
où
Y représente un radical polyéther divalent du type - (CH₂-CHR²-O)ₘ-CH₂-CHR²- et m représente un nombre entier de 0 à 50 et R² représente hydrogène ou un groupe alkyle comprenant 1-4 atomes de carbone ;
C représente un fragment de la structure amine des amines disecondaires choisies parmi la pipérazine, la N,N'-diméthylhexaméthylène-1,6-diamine et la N,N'-diméthyltétraméthylène-1,4-diamine,
x représente un nombre de 0,1 à 10,
y représente un nombre de 1,1 à 50,
pouvant être obtenus par la transformation d'organopolysiloxanes fonctionnalisés par des groupes aminoalkyle secondaires avec les produits de réaction de composés contenant des groupes époxyde et d'amines.

2. Procédé pour la préparation de copolymères linéaires de polydiméthylsiloxane-polyéther présentant des groupes amino et/ou d'ammonium quaternaire, **caractérisé en ce que**
a) on transforme d'abord un diépoxyde avec au moins un composé amine en un produit d'addition à chaîne allongée portant des groupes glycidyle en position terminale, le rapport molaire de diépoxyde à amine étant variable mais au moins supérieur à 1:1, et
b) on fait ensuite réagir ce produit intermédiaire avec un polysiloxane linéaire portant des groupes amino secondaires terminaux,
c) des acides ou des réactifs d'alkylation peuvent éventuellement être ajoutés en un endroit quelconque dans ce procédé pour la quaternisation complète ou partielle des atomes d'azote, et
d) on ajoute en outre éventuellement de l'eau ou des composés organiques, choisis dans le groupe des polyéthers, des polyols ou des alcools comme diluants, émulsifiants ou agents de modification.

3. Procédé selon la revendication 2, **caractérisé en ce qu'**on utilise, comme diépoxydes, des composés de formule (1) 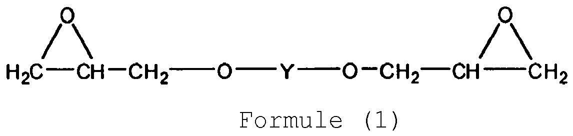 où
Y représente un radical polyéther divalent du type - (CH₂-CHR²-O)ₘ-CH₂-CHR²- et m représente un nombre entier de 0 à 50 et R² représente hydrogène ou un groupe alkyle comprenant 1-4 atomes de carbone.

4. Procédé selon au moins l'une quelconque des revendications 2 ou 3, **caractérisé en ce qu'**on utilise comme composés amine de l'étape de procédé (a) des amines disecondaires choisies parmi la pipérazine et/ou la N,N'-diméthylhexaméthylène-1,6-diamine et/ou la N,N'-diméthyltétraméthylène-1,4-diamine.

5. Procédé selon au moins l'une quelconque des revendications 2 à 4, **caractérisé en ce qu'**on utilise, comme composés de l'étape de réaction (b), des polysiloxanes présentant des groupes amine secondaires terminaux selon la formule (3) 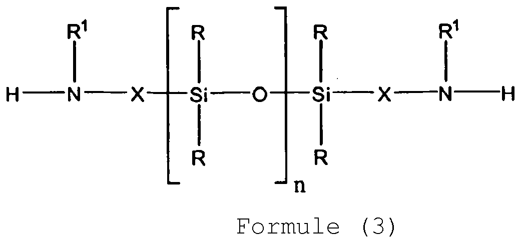 où
R représente un groupe méthyle,
R¹ représente un quelconque radical hydrocarboné,
X représente un radical hydrocarboné divalent, linéaire ou ramifié, comprenant 1-20 atomes de carbone, qui peut être interrompu par des atomes d'azote ou des groupes aminiques, et
n représente un nombre entier de 1 à 500.

6. Procédé pour la préparation de composés selon au moins l'une quelconque des revendications 2 à 5 contenant des groupes d'ammonium quaternaire, **caractérisé en ce qu'**on ajoute un acide ou un réactif d'alkylation dans l'étape de procédé (c).

7. Copolymères de formule (4) selon la revendication 1 et leurs dérivés quaternisés par des acides et/ou des agents d'alkylation, portant des groupes d'ammonium.

8. Compositions contenant les copolymères selon les revendications 1 ou 7, concentrats, mélanges/concentrats en émulsion les contenant et/ou leurs formulations aqueuses, émulsions aqueuses et/ou solutions, formulation ou émulsion dans des composés organiques tels que des polyéthers, des polyols, des alcools.

9. Utilisation des copolymères selon les revendications 1 ainsi que 7 à 8, pouvant être obtenus selon au moins un des procédés des revendications 2 à 6 comme plastifiant permanent pour des structures planes choisies dans le groupe comprenant les tissus, les tissus textiles, les étoffes, les tricots, les non-tissés, les lingettes, les fibres de papier ; et/ou les fibres de matières premières et/ou de cuir et/ou de poils et/ou de peaux, naturels et/ou synthétiques.

10. Utilisation selon la revendication 9, **caractérisée en ce que** le plastifiant confère des propriétés hydrophiles aux structures planes traitées par celui-ci.

11. Utilisation selon au moins l'une quelconque des revendications 9 ou 10 dans des formulations, **caractérisée en ce que** l'aptitude au peignage, la souplesse, le volume, l'aptitude à la mise en forme, la maniabilité, l'aptitude au démêlage de cheveux non abîmés et abîmés sont améliorés et/ou de la brillance est conférée aux cheveux.

12. Utilisation selon au moins l'une quelconque des revendications 9 à 11 dans des mélanges, contenant les copolymères de formule générale (4) ou les compositions, dans des formulations de soins cosmétiques améliorant la brillance, comme agents de traitement et de post-traitement de cheveux pour le rinçage ou pour rester dans les cheveux, agents de conditionnement, shampooings 2-en-1, produits de rinçage, cures pour cheveux, masques pour cheveux, adjuvants de coiffage, produits de mise en pli, lotions de séchage, fixateurs, agents de permanente, agents de lissage des cheveux et/ou agents pour la teinture des cheveux.

13. Utilisation selon au moins l'une quelconque des revendications 9 à 10 dans des mélanges, contenant les copolymères de formule générale (4) ou les compositions, pour des formulations cosmétiques, dermatologiques et pharmaceutiques ainsi que des agents cosmétiques de soin et de nettoyage du corps/visage, contenant au moins un composant supplémentaire choisi dans le groupe comprenant les émollients, les émulsifiants et les tensioactifs, les épaississants/régulateurs de viscosité/stabilisateurs, les filtres de protection contre la lumière UV, les antioxydants, les hydrotropes ou les polyols, les solides et les charges, les agents filmogènes, les additifs nacrés, les substances actives déodorantes et antiperspirantes, les répulsifs d'insectes, les autobronzants, les conservateurs, les agents de conditionnement, les parfums, les colorants, les substances actives cosmétiques, les additifs de soin, les agents graissants, les solvants.

14. Utilisation selon au moins l'une quelconque des revendications 9 à 10 ainsi que 13 dans des mélanges, contenant les copolymères de formule générale (4) ou les compositions, pour des utilisations cosmétiques sous forme d'une formulation aqueuse, tensioactive pour obtenir une sensation agréable, veloutée-soyeuse sur la peau.
